# EUROPEAN PATENT APPLICATION

(11) **EP 3 827 854 A1**
(43) Date of publication of application: **02.06.2021**
(21) Application number: 20209961.0
(22) Date of filing: 26.11.2020
(51) Int. Cl.: A61M 5/14, A61M 5/142, A61M 5/145, A61M 5/148, A61M 5/168, A61M 5/172

(54) **DEVICE FOR THE ADMINISTRATION OF LIQUIDS, DRUGS OR NUTRIENTS TO A PATIENT**

(30) Priority: 27.11.2019 IT 201900022281
(71) Applicant: Fluid-O-Tech S.r.l., 20094 Corsico (MI) (IT)
(72) Inventor: ANDREIS, Diego, 20145 Milano (IT); BARNI, Greta, 20020 Arese (MI) (IT); PALMIERI, Stefania, 20145 Milano (IT); FEBBRARI, Margherita, 25060 Cellatica (BS) (IT)
(74) Representative: Frasson, Luca

(57) **Abstract**

The present invention relates to a device (1) for the administration of liquids, drugs or nutrients to a patient (P) comprising a deformable bag (3) for containing a liquid or a drug or a nutrient to be administered to a patient (P), in fluid communication, through a tube (5), with a needle (7) or a tube or a catheter that can be inserted in a patient (P).

According to the invention, the device (1) comprises:
- an expandable air container (9) whose expansion generates the compression of said deformable bag (3);
- compressed air generation means (11) adapted to introduce air into said expandable air container (9),
- a first proportional valve (13) associated with said tube (5) and configured to regulate the flow of said liquid or drug or nutrient from said deformable bag (3) generated by said compression of said deformable bag (3) due to said expansion of said expandable air container (9),
- a control unit (15) connected to said first proportional valve (13) configured to control the operation of said first proportional valve (13) according to parameters of administration of said liquid or drug or nutrient to said patient (P).

## Description

The present invention relates to a device for the administration of liquids, drugs or nutrients to a patient.

There currently exist several and different medical conditions in which it is provided to administer liquids to a patient.

A first example relates to intravenous infusion of liquids, such as saline solutions, or drugs, for instance in form of therapeutic solutions. Another example relates to blood transfusion. Still another example relates to artificial nutrition, which requires the administration to a patient of nutrients in liquid form, through the gastroenteric apparatus, called enteral nutrition, or through a vein, called parenteral nutrition.

The most commonly used devices for intravenous infusion comprise a bag or a bottle containing the liquid to be administered, a tube for intravenous drip and a needle inserted in the patient's vein. As is known, such devices operate by gravity and therefore do not require any energy consumption to operate.

There also currently exist devices which provide an energy consumption to operate, as they comprise a pump able to transmit energy to the liquid to be administered to the patient.

Such pump devices are mainly employed in the field of artificial nutrition.

The aforementioned devices of the known type, both those operating by gravity and those operating by means of pumps, have some drawbacks among which the fact that they do not allow a consistent and accurate administration of the liquid, drug or nutrient to the patient.

Another drawback of such devices of the known type consists in that they are bulky and provoke significant discomfort to the patient using them, as they prevent, or in any case limit dramatically, free movements.

The task of the present invention consists in making a device for the administration of liquids, drugs or nutrients to the patient that solves the drawbacks and overcomes the limits of the prior art allowing for a consistent and accurate administration of the liquid, drug or nutrient to the patient.

Within this task, an object of the present invention is to make a device that is compact, made in a single piece and light.

Another object of the invention consists in making a device that has a long autonomy when used.

Another object of the invention consists in making a device that can be used also in emergency situations, such as in non-equipped places.

Another object of the invention consists in making a device whose operation may be also remote-controlled.

A further object of the invention consists in making a device that is able to provide the broadest guarantees of reliability and security when used.

Another object of the invention consists in making a device that is easy to make and use and altogether cost-effective when compared with the prior art.

The task set forth above, as well as the mentioned objects and others that will better appear hereinafter, are obtained by a device for the administration of liquids, drugs or nutrients to a patient as claimed in claim 1.

Other features are comprised in the dependent claims.

Additional features and advantages will become more apparent from the description of two preferred, but non-exclusive, embodiments of a device for the administration of liquids, drugs or nutrients to a patient, illustrated by way of non-limiting examples with the aid of the appended drawings, in which:
figure 1 is a schematic representation of the hydraulic circuit of a first embodiment of a device for the administration of liquids, drugs or nutrients to a patient, according to the invention;
figure 2 is a perspective view of a first embodiment of the device for the administration of liquids, drugs or nutrients to a patient, according to the invention;
figure 3 is an exploded perspective view of the device of figure 2;
figures 4 and 5 are two exploded perspective views of two components of the device of figure 2;
figure 6 is a further exploded perspective view of the device of figure 2;
figure 7 schematically illustrates a patient wearing the device of figure 2;
figure 8 is a schematic representation of a second embodiment of the device for the administration of liquids, drugs or nutrients to a patient, according to the invention;
figure 9 is a block diagram which illustrates the operation of the device for the administration of liquids, drugs or nutrients to a patient, according to the invention;
figures 10 to 12 illustrate three sectional views of three different variants of a part of the device for the administration of liquids, drugs or nutrients to a patient, according to the invention;
figure 13 is a sectional view of the components represented in figure 12, made according to the axis XIII-XIII.

With reference to the mentioned figures, the device for the administration of liquids, drugs or nutrients to a patient, referred to as a whole by number 1, comprises a deformable bag 3 containing a liquid or a drug or a nutrient to be administered to a patient P, in fluid communication, through a tube 5, with a needle 7 or a tube or a catheter that can be inserted in the patient P.

According to the invention, the device 1 comprises:
- an expandable air container 9 whose expansion generates the compression of said deformable bag 3,
- compressed air generating means 11 adapted to introduce air into said expandable air container 9,
- a first proportional valve 13 associated with said tube 5 and configured to regulate the flow of said liquid (or drug or nutrient) from the deformable bag 3 generated by the compression of the deformable bag 3 due to the expansion of the expandable air container 9,
- a control unit 15 connected to said first proportional valve 13 configured to control the operation of said first proportional valve 13 according to parameters of administration of the liquid (or drug or nutrient) to the patient P.

Advantageously the presence of the first proportional valve 13 for regulating the flow of the liquid from the deformable bag 3 allows to control and vary the flow rate of the liquid exiting from the deformable bag 3 and thus to control and vary the administration thereof to the patient P.

Advantageously the device 1 comprises a second proportional valve 17 configured to regulate the air flow from compressed air generating means 11 to the expandable air container 9. Also the second proportional valve 17 is connected to the control unit 15, which is configured to control also the operation of such second proportional valve 17.

Advantageously the presence of the second proportional valve 17 allows to control and vary the flow rate of the air entering the expandable air container 9 and allows indirectly, accordingly, to control and vary the compression of the deformable bag 3, and finally allows to contribute to control and vary the flow rate of the liquid exiting from the deformable bag 3.

Advantageously the first proportional valve 13 and/or the second proportional valve 17 comprise a valve element which can be actuated through an actuator comprising an active element made in a shape memory material and configured to generate a proportional opening and closing movement of the valve element depending on its shape and/or dimensions.

Advantageously the active element made in a shape memory material has an electric resistivity dependent on the variations of shape and/or size of the active element, where such electric resistivity variations can be used for the closed-loop control of the actuation of the active element.

Advantageously the active element consists in a thread made of a shape memory material. Advantageously the compressed air generating means 11 comprise a compressed air cartridge 110, preferably associated with a pressure reducer 111, or an air pump 112.

Advantageously the device 1 may comprise a first flow sensor 14 placed downstream of the first proportional valve 13 and adapted to measure the flow rate of the liquid (or drug or nutrient) exiting from the first proportional valve 13. Such first flow sensor 14 is in data communication with the control unit 15 and allows as such a feedback control of the operation of the first proportional valve 13.

Advantageously the device 1 comprises a second flow sensor 18 placed downstream of the second proportional valve 17 and adapted to measure the flow rate of the air exiting from the second proportional valve 17. Such second flow sensor 18 is in data communication with the control unit 15 and allows as such a feedback control of the operation of the second proportional valve 17.

Advantageously the device 1 comprises a temperature sensor adapted to detect the temperature of the liquid or drug or nutrient to be administered to the patient P, where such temperature sensor is in data communication with the control unit 15.

Advantageously the device 1 may comprise a first temperature sensor 16 adapted to detect the pressure of the liquid or drug or nutrient to be administered to the patient P, where such pressure sensor is in data communication with the control unit 15.

Such pressure sensor 16 may be arranged upstream and/or downstream of the first proportional valve 13 in order to detect the pressure of the liquid or drug or nutrient to be administered to the patient P.

In alternative, or in addition, a second pressure valve 18' may also be provided upstream and/or downstream of the second proportional valve 17, to detect the air pressure entering the compressed air container 9.

The possibility to detect the pressure of the liquid or air upstream and/or downstream of the proportional valves 13 and 17, and preferably downstream thereof, allows to accurately control the operation of administration and/or infusion of the liquid (or drug or nutrient).

Advantageously the control unit 15 comprises a data transceiver module 150 configured to receive and transmit data by a wireless communication with a personal computer 151 and/or with a smartphone 152 and/or with similar devices.

Advantageously the control unit 15 also comprises a data processing electronic circuit, such as a CPU (Central Processing Unit) or a MCU (Micro Controller Unit) and a programmable memory card.

Advantageously the device 1 is supplied by batteries 20, preferably rechargeable, present in the device 1.

The device 1 may also have a screen 21 in which a plurality of information may be viewed such as the level (for instance in terms of volume) of liquid present in the deformable bag 3, the level of electric charge of the batteries 20, the set dosage of administration of the liquid (or drug or nutrient), for example in ml/s or in 1/min and, for instance, in case a cartridge of compressed air 110 is present, the level of compressed air present in the cartridge 110, as well as the temperature of the liquid (or drug or nutrient), or the pressure thereof.

Advantageously it is also possible to make available through the screen 21 further possible functionalities such as signalling malfunctions of the device and/or abnormal parameter values.

Advantageously the device 1 may have a user interface through which it is possible to set for instance the operation parameters of the first proportional valve 13 and/or second proportional valve 17.

Preferably the screen 21 is a touch screen serving as a user interface for controlling the operation of the device 1.

Not only is it possible to continuously and immediately monitor through the screen 21 the operation of administration and/or infusion of the liquid (or drug or nutrient), but also to act on the control thereof.

In alternative, or in addition, the operation parameters of the first proportional valve 13 and/or of the second proportional valve 17 may be controlled by the personal computer 151 and/or the smartphone 152.

Advantageously all the information available on the screen 21 may be provided in alternative or in addition also to the personal computer 151 and/or smartphone 152.

Advantageously in the programmable memory card present in the control unit 15, the operation parameters of the device 1 may be stored, and in particular of the first proportional valve 13 and/or second proportional valve 17, during a treatment. Furthermore in the programmable memory card also the signals detected by the sensors present in the device 1 during a treatment may be stored, such as the signals detected by the first flow sensor 14, and/or by the second flow sensor 18, and/or by the temperature sensor, and/or by the first pressure sensor 16, and/or by the second pressure sensor 18'.

Advantageously the expandable air container 9 is made of an elastomeric material configured to elastically return to its rest position when it is not filled with pressurized air.

Advantageously also the deformable bag 3 containing the liquid, drug or nutrient to be administered to a patient P may be made in an elastomeric material configured to elastically return to its rest condition when not submitted to an external pressure.

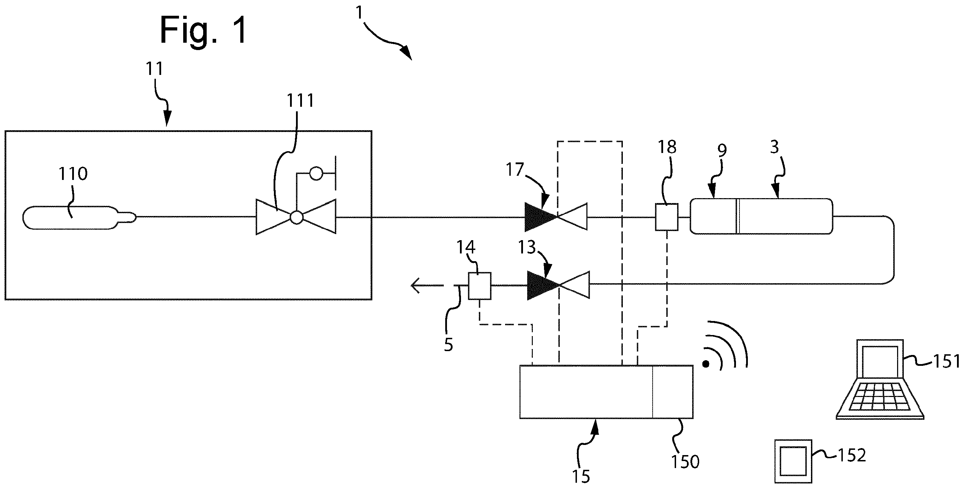

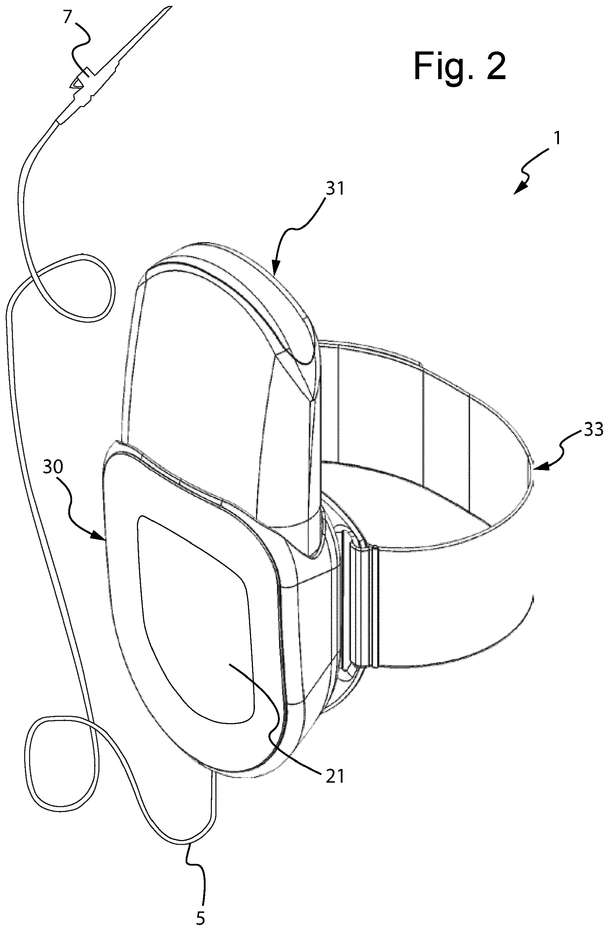

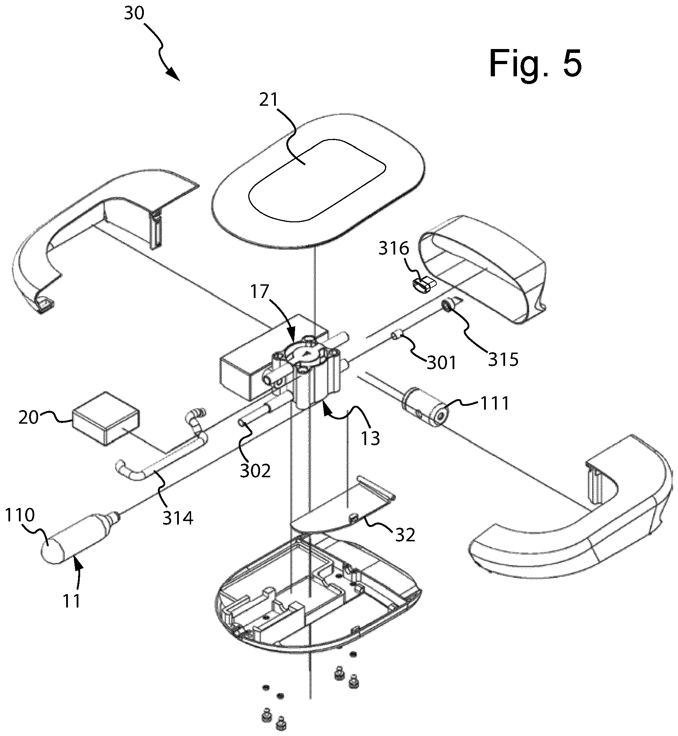

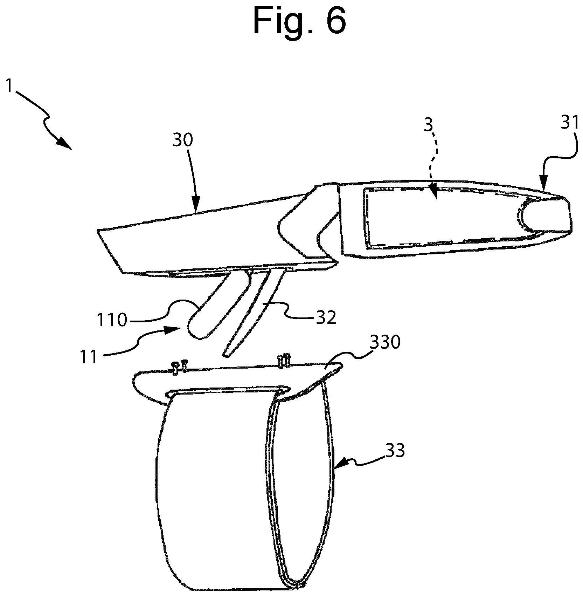

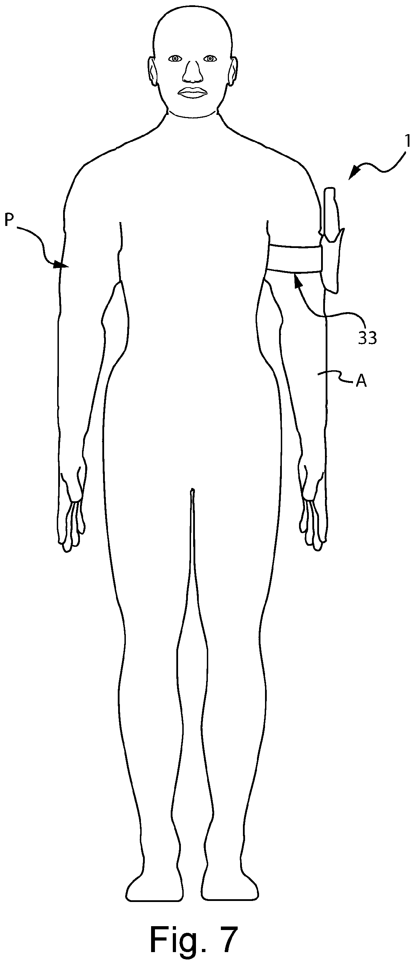

A first embodiment of the device 1, that is particularly suitable for the intravenous infusion of an infusion liquid or of an infusion drug is illustrated in figures 1 to 7.

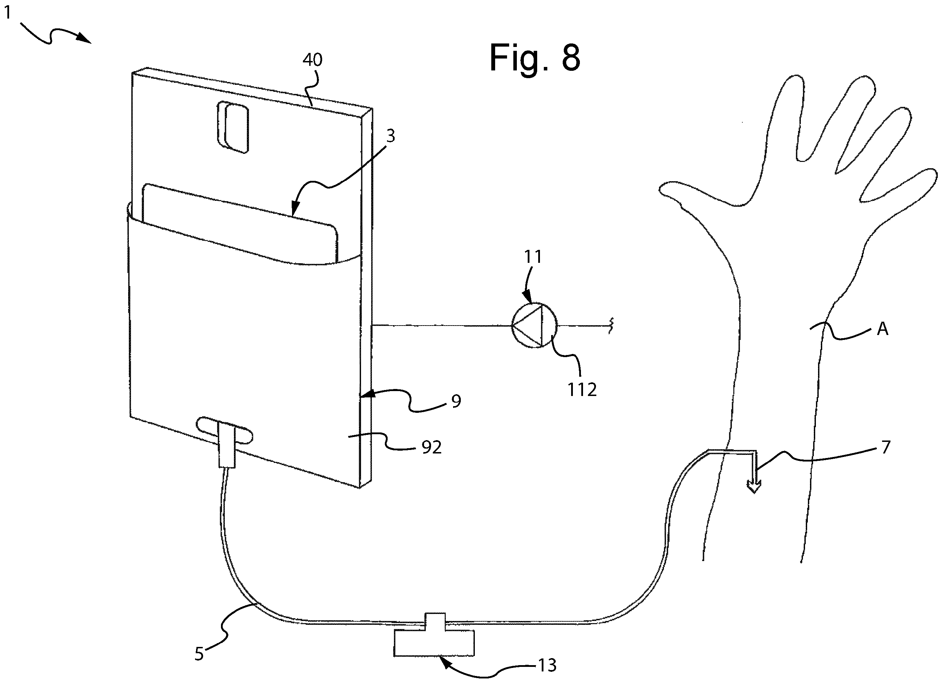

Figure 8 illustrates instead a second embodiment of the device 1, that is particularly suitable for the enteral artificial nutrition, i.e. through the gastroenteric apparatus, and/or parenteral artificial nutrition, i.e. through a vein.

In case of intravenous infusion, the device 1 advantageously comprises a rigid container 31 housing therein both the deformable bag 3 and the expandable air container 9. The expansion of such air container 9 generates the compression of the deformable bag 3 housed inside the rigid container 31.

Advantageously the expandable air container 9 is a balloon 90.

Advantageously the device 1 also comprises a rigid casing 30 containing internally at least the compressed air generating means 11, the first proportional valve 13 and the control unit 15. The rigid casing 30 is associable with the aforesaid rigid container 31 so that compressed air generating means 11 are in fluid communication with the expandable air container 9 and that the deformable bag 3 is in fluid communication with the first proportional valve 13.

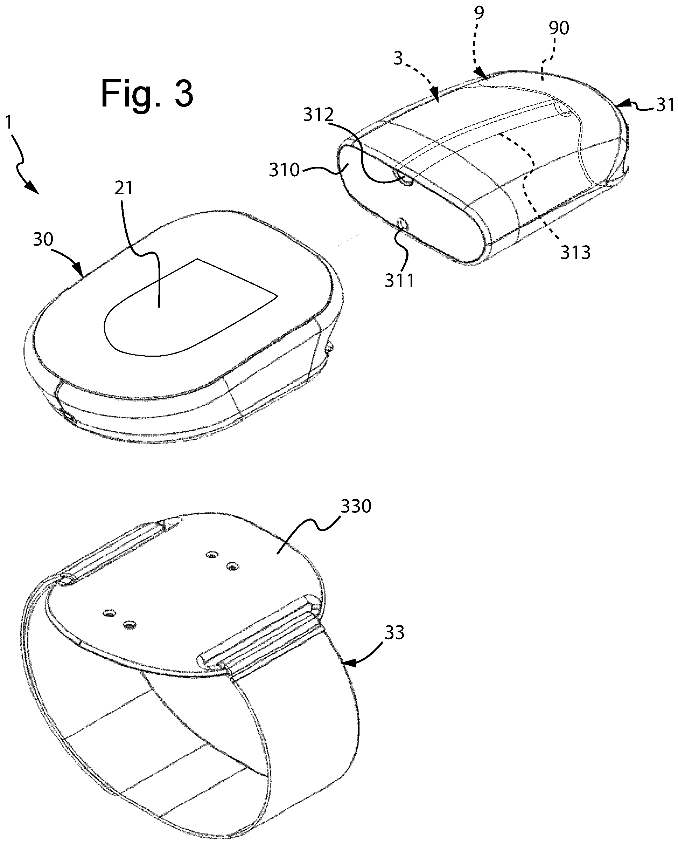

Substantially, as illustrated in particular in figures 3, 4 and 5, the rigid container 31 has a base 310 intended, on the outer side, to be connected to the rigid casing 31. The deformable bag 3 is instead in contact with the inner side of the base 310.

The base 310 also has a first hole 311 adapted to be passed through from a first piercing element 315 present in the rigid casing 30, such that the deformable bag 3 is housed inside the rigid container 31 and this is hooked to the rigid casing 30, the piercing element 315 passes through the first hole 311 and, by piercing the deformable bag 3, puts in fluid communication the content of the deformable bag 3 with the first proportional valve 13, through the duct 301 and thus, through the duct 302 with the tube 5 to the patient P.

Advantageously the rigid container 31 also comprises a lid 317, preferably removable to be able to access the expandable air container 9, for example for maintenance purposes.

Advantageously the rigid container 31 may house therein also deformable bags 3 of the known type, such as those commonly used in hospitals.

Advantageously the compressed air generating means 11 comprise a compressed air cartridge 110 housed inside the rigid casing 30. Such rigid casing 30 advantageously comprises an openable door 32 adapted to allow access to said compressed air cartridge 110 for replacement thereof.

Advantageously the rigid container 31 comprises a duct 313 which connects a second hole 312 present on the base 310 with the expandable air container 9.

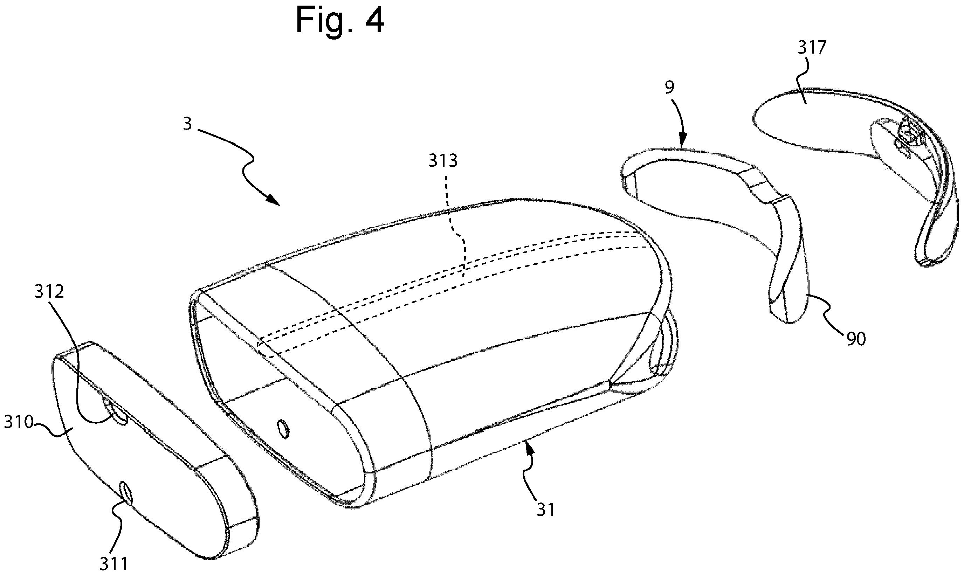

Advantageously, as illustrated in figures 3 and 4, such duct 313 is obtained adjacent to an inner wall of the rigid container 31.

When the rigid container 31 is fixed to the rigid casing 30, the duct 313 is put in fluid communication, through the second hole 312, thanks to a second piercing element 316, and through the duct 314, with the compressed air cartridge 110, also possibly through the pressure reducer 111.

Advantageously, in particular in case the device 1 is adapted to contain a deformable bag 3 containing infusion liquid or infusion drug to be administered to the patient P intravenously, the device 1 also comprises a fixing band 33, preferably made of elastic silicon, for fixing the device 1 to a limb A of the patient P, such as an arm.

Advantageously the fixing band 33 also comprises a rigid base 330 which the rigid casing 30 can be fastened to, for instance by reversible fixing means.

The device 1 is advantageously able to operate properly in any position and orientation taken by the patient arm P.

In figure 8 the second embodiment of the device 1 which refers in particular to a device for artificial nutrition, is schematically illustrated.

In this case the expandable air container 9 advantageously comprises an inflatable pocket 92, within which the deformable bag 3 can be inserted.

Such inflatable pocket 92 may be associated with a support structure 40, preferably at least partially rigid.

Advantageously such support structure 40 may be associated with straps or shoulder straps or cross body straps which allow the patient P to bring the device 1 along.

Compressed air generating means 11 advantageously consist of an air pump 112.

By actuating the air pump 112 the inflatable pocket 92 inflates, compressing the deformable bag 3 contained therein and thus determining nutrients contained inside the deformable bag 3 to flow to the patient P.

Even in this case the deformable bag 3 may consist of a bag for artificial nutrition of the known type and commonly used both in hospitals and at home.

Advantageously the device 1 may have the shape of a rucksack apparently similar to rucksacks of the known type.

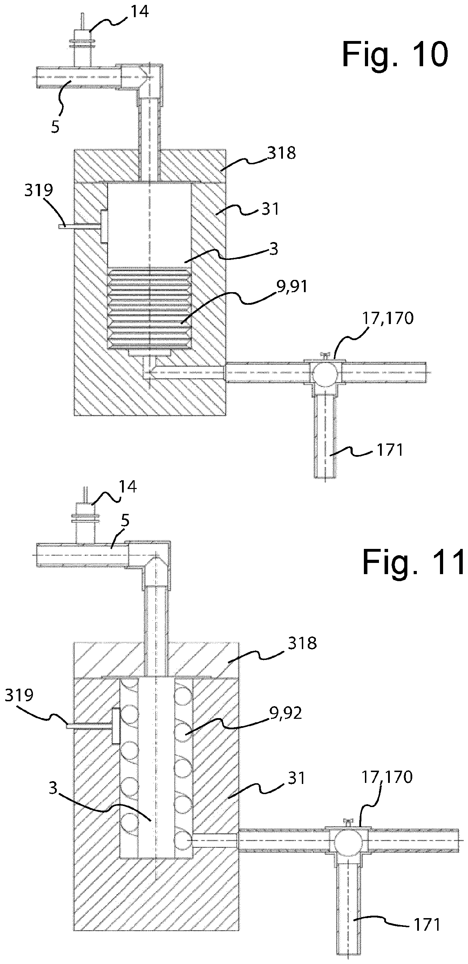

In figures 10 to 13 three different variants of the mechanism adapted to compress the deformable bag 3 containing the liquid, the drug or the nutrient to be administered to the patient are schematically illustrated.

With reference to all the three aforesaid variants, the device 1 comprises a rigid container 31, housing therein, both the deformable bag 3 and the expandable air container 9, where the expansion of the air container 9 involves the compression of the deformable bag 3 housed inside the rigid container 31. Advantageously a removable lid 318 may be provided to access both the deformable bag 3 and the expandable air container 9, for instance for maintenance purposes thereof. Advantageously, the device 1 further comprises a capacitive sensor 319 adapted to generate a signal indicative of the level of liquid, drug or nutrient inside the rigid container 31, which signal can be sent to the control unit 15.

Advantageously, as illustrated in figures 10 to 13, the proportional valve 17 is preferably a three-way valve 170 configured to regulate the air flow from compressed air generating means 11 to the expandable air container 9 through a discharge way 171.

Furthermore, in figures 10 to 12 the flow sensor 14 applied to the tube 5 is illustrated. Advantageously such flow sensor 14 is a flow rate sensor of the optic type, such as for example a drip sensor. Preferably such flow sensor 14 is applied downstream of the first proportional valve 13.

As illustrated in figure 10, in one first variant, the expandable air container 9 comprises a container configured as a bellows 91. Advantageously the sidewalls of such container 9 have indeed a bellows structure adapted to allow, inside the rigid container 31, the elastic expansion of the inner volume to such container 9 due to the elongation of the bellows sidewalls.

As illustrated in figure 11, in a second variant, the expandable air container 9 comprises a helicoidal-shaped bag 92 with known non-expanded volumes which wraps the deformable bag 3 containing the liquid or drug or nutrient. The increase in volume of the helicoidal-shaped bag 92 inside the rigid container 31 generates the compression of the deformable bag 3.

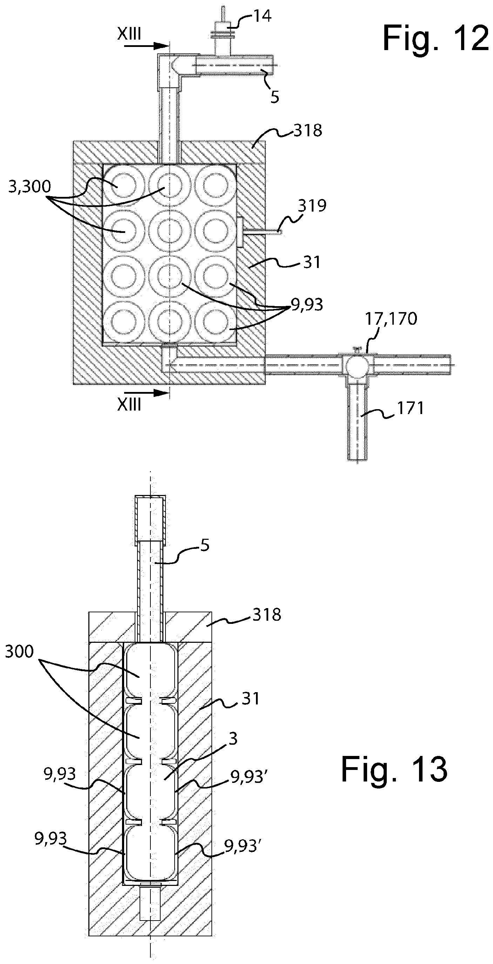

As illustrated in figures 12 and 13, in a third variant, the deformable bag 3 containing the liquid or drug or nutrient comprises a plurality of mutually communicating spheres 300, while the expandable air container 9 comprises a plurality of pairs of hollow half-spheres 93, 93' mutually communicating and configured to wrap, in pairs, in a substantially complimentary way, each one of the spheres 300. Thereby the increase in the volume of the hollow half-spheres 93, in fluid communication between each other, generates the compression of the spheres 300, that are also in fluid communication between each other, and therefore the compression of the deformable bag 3 in its entirety.

Advantageously the deformable bag 3 containing the liquid or drug or nutrient may be made in a thermostable and/or electrically conductive polymer in order to make the administration more stable and repeatable, in particular in the typical case of fluids whose viscosity varies sensibly according to the temperature. Advantageously the deformable bag 3 may comprise flexible ultra-flat electrical resistances that are coupled to the polymeric material of which the deformable bag 3 is made, actuatable by the control unit 15 to keep the temperature of the liquid or drug or nutrient contained in the deformable bag 3 itself.

The operation of the device for the administration of liquids, drugs or nutrients to a patient is clear and apparent from what above described, both with reference to the first embodiment, related to intravenous infusion, and with reference to the second embodiment, related to artificial nutrition.

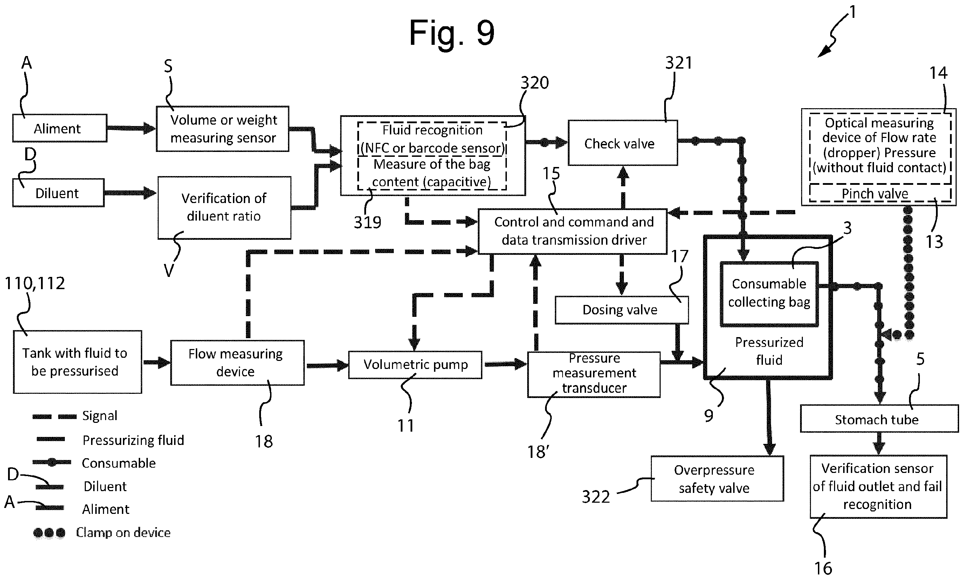

In figure 9 a block diagram is illustrated showing the operation of the device 1 for administering liquids, drugs or nutrients to a patient, with particular reference to an administration example, wherein an aliment A is mixed with a diluent D to form a nutrient, also called "consumable", to be delivered to the patient P.

The block diagram, and the following description, is also applicable in case of drug administration, where for example a desired drug, in liquid or solid form, must be diluted into a given diluent.

To form the nutrient, a given amount of aliment A, which can be measured through a weight and/or volume measuring sensor S, is diluted with a given amount of diluent D, upon due verification V of the aliment/diluent ratio.

The device 1 then advantageously comprises a system for recognising 320 the nutrient (or the liquid, or the drug) which is going to be delivered to the patient. Indeed the device 1 may comprise a NFC (Near Field Communication) sensor, or a bar code reader or a QR code reader, that are able to exchange data, directly or indirectly, with the control unit 15, in order to control the correctness of the selected nutrient (or liquid, or drug) to be administered to the patient P.

The presence of a capacitive sensor 319 further allows to know in real time the level of nutrient (or liquid, or drug) present in the deformable bag 3.

Advantageously, upstream of the deformable bag 3, the device 1 also comprises a check valve 321.

Then when the nutrient (or liquid, or drug) is present inside the deformable bag 3, it is possible by means of the expandable air container 9 to proceed administering the nutrient to the patient P. Such administration is controlled through the first proportional valve 13, also named "pinch valve" in the block diagram of figure 9, as it can operate by narrowing, like a pinch, the deformable walls of a channel through which the nutrient flows.

Furthermore the presence of the sensor 14 measuring the flow rate of the nutrient, and possibly the presence of the pressure sensor 16 of the nutrient, allows the control unit 15 to control the operation, at least, of the first proportional valve 13 and thus to control the administration of the nutrient of the patient, as well as to detect the presence of malfunctions and/or undesired interruptions in administering the nutrient, in order to activate possible alerts.

Furthermore, it is also possible to act on the second proportional valve 17, to adjust the air flow intended to inflate the expandable air container 9. The presence of the flow sensor 18 and/or of the pressure sensor 18' at the line of the pressurizing fluid allows to adjust in real time and in a very accurate way the operation of compressed air generating means 11, and consequently to vary the administration of the nutrient (or liquid, or drug) in addition to what operated by the first proportional valve 13.

Advantageously, the device 1 also comprises an overpressure safety valve 322 configured to prevent an excessive and undesired rise in the air pressure inside the expandable air container 9.

It was in fact proven than the device, according to the present invention, fulfils the task as well as preset objects as it allows to administer liquids, drugs or nutrients to a patient in a constant, stable and accurate way.

Another advantage of the device, according to the invention, consists in that it is portable.

In fact the device, according to the invention, both in case of intravenous infusion application and in case of artificial nutrition application, may be worn by the patient, so that the patient is completely free to move.

A further advantage of the device according to the invention, consists in that it does not require any difference in height and related support bases to operate.

Another advantage of the device, according to the invention, consists in that it is particularly compact, made in a single piece and light.

Another advantage of the device, according to the invention, consists in that it can ensure a high autonomy.

A further advantage of the device, according to the invention, consists in that it can be remote-controlled, by a personal computer or a smartphone, or still by an interface present on the device. It is thereby possible to vary the administration of the liquid, or drug, or nutrient in an absolutely versatile way. It is further possible to store the administration parameters of the liquid, drug or nutrient and create a history of the administrations performed, so as to improve monitoring the treatment over time.

Still another advantage of the device, according to the invention, consists in that it can be used in any situation, and in particular in critical emergency situations such as road emergency services, helicopter rescue, alpine rescue, etcetera.

A further advantage of the device, according to the invention, consists in that the administration of liquids, drugs or nutrients can be controlled in a precise and prompt way regardless of the positioning and orientation of the device itself.

Still another advantage of the device, according to the invention, and in particular of the device for intravenous infusion, consists in that the compressed air cartridge and/or bag containing the liquid or infusion liquid can be easily replaced, and can thus reduce as much as possible the time range while the device is not operative.

Still another advantage of the device, according to the invention, consists in that the presence of a plurality of valves and sensors allows to increase the control redundancy of the device, and consequently to ensure the reliability and safety of the device operation.

In particular, thanks to the fact that it can detect and store the operation parameters of the device 1, and consequently the parameters relative to a specific treatment session, it is possible to store a history of the treatments, which can be used for example to vary the parameters of the treatments over time, for instance enabling the physician or the healthcare operator, to optimise and adjust the treatment, both during a single session, and between one session and the other, upon alerts, malfunctions or care optimization being necessary, all of which also possibly remotely. Furthermore the device enables the physician or the healthcare operator to ascertain the patient adherence to the treatment, in particular for those treatments that are performed at home, without the physician or healthcare operator.

The device for the administration of liquids, drugs or nutrients to a patient as conceived is susceptible to several modifications and variants all falling within the scope of the inventive concept.

Furthermore, all the details can be replaced by other technically equivalent elements.

In practice, any materials can be used according to requirements, as long as they are compatible with the specific use, the dimensions and the contingent shapes.

## Claims

1. Device (1) for the administration of liquids, drugs or nutrients to a patient (P) comprising a deformable bag (3) for containing a liquid or a drug or a nutrient to be administered to a patient (P), in fluid communication, through a tube (5), with a needle (7) or a tube or a catheter that can be inserted in a patient (P), **characterized in that** it comprises:
- an expandable air container (9) whose expansion generates the compression of said deformable bag (3);
- compressed air generating means (11) adapted to introduce air into said expandable air container (9);
- a first proportional valve (13), associated with said tube (5), and configured to regulate the flow of said liquid or drug or nutrient from said deformable bag (3) generated by said compression of said deformable bag (3) due to said expansion of said expandable air container (9);
- a control unit (15) connected to said first proportional valve (13) configured to control the operation of said first proportional valve (13) as a function of parameters of administration of said liquid or drug or nutrient to said patient (P).

2. Device (1), according to claim 1, **characterized in that** it comprises a second proportional valve (17) configured to regulate the flow of air from said compressed air generating means (11) to said expandable air container (9), said second proportional valve (17) being connected to said control unit (15), said control unit (15) being configured to control the operation of said second proportional valve (17).

3. Device (1) according to claim 1 or 2, **characterized in that** said first proportional valve (13) and/or said second proportional valve (17) comprise a valve element which can be actuated through an actuator comprising an active element made in a shape memory material and configured to generate a proportional opening and closing movement of said valve element depending on its shape and/or dimensions.

4. Device (1) according to claim 3, **characterized in that** said active element made of a shape memory material has an electrical resistivity dependent on the variations of shape and/or size of the active element itself, wherein said variations of electrical resistivity can be used for the closed-loop control of the activation of said active element.

5. Device (1) according to one or more of the preceding claims, **characterized in that** said compressed air generating means (11) comprises a compressed air cartridge (110), preferably associated with a pressure reducer (111), or an air pump (112).

6. Device (1) according to one or more of the preceding claims, **characterized in that** it comprises a first flow sensor (14) placed downstream of said first proportional valve (13) and adapted to measure the flow rate of said liquid or drug or nutrient exiting from said first proportional valve (13), said first flow sensor (14) being in data communication with said control unit (15).

7. Device (1) according to one or more of claims 2 to 6, **characterized in that** it comprises a second flow sensor (18) located downstream of said second proportional valve (17) and adapted to measure the flow rate of said air exiting from said second proportional valve (17), said second flow sensor (18) being in data communication with said control unit (15).

8. Device (1) according to one or more of the preceding claims, **characterized in that** it comprises a temperature sensor adapted to detect the temperature of said liquid or drug or nutrient to be administered to said patient (P), said temperature sensor being in data communication with said control unit (15).

9. Device (1) according to one or more of the preceding claims, **characterized in that** it comprises at least one first pressure sensor placed upstream and/or downstream of said first proportional valve (13) adapted to detect the pressure of said liquid or drug or nutrient to be administered to said patient (P), said at least one first pressure sensor being in data communication with said control unit (15).

10. Device (1) according to one or more of the claims 2 to 9, **characterized in that** it comprises at least one second pressure sensor positioned upstream and/or downstream of said second proportional valve (17) adapted to detect the pressure of said air flowing from said compressed air generating means (11) to said expandable air container (9), said at least one second pressure sensor being in data communication with said control unit (15).

11. Device (1) according to one or more of the preceding claims, **characterized in that** it comprises a rigid container (31) containing internally said deformable bag (3) and said expandable air container (9), wherein the expansion of said expandable air container (9) generates the compression of said deformable bag (3) inside said rigid container (31).

12. Device (1) according to claim 11, **characterized in that** it comprises a rigid casing (30) containing internally at least said compressed air generation means (11), said first proportional valve (13) and said control unit (15), said rigid casing (30) being associable with said rigid container (31) so that said compressed air generating means (11) are in fluid communication with said expandable air container (9) and that said deformable bag (3) is in fluid communication with said first proportional valve (13).

13. Device (1), according to claim 12, **characterized in that** said compressed air generation means (11) comprise a compressed air cartridge (110) housed in said rigid casing (30), said rigid casing (30) comprising an openable door (32) adapted to allow access to said compressed air cartridge (110) for replacement thereof.

14. Device (1) according to one or more of the preceding claims, **characterized in that** said deformable bag (3) contains an infusion liquid or infusion drug to be administered to said patient (P) intravenously, said device (1) comprising a fixing band (33) for fixing said device (1) to a limb (A) of said patient (P).

15. Device (1), according to one or more of the claims 1 to 13, **characterized in that** said deformable bag (3) contains a nutritional liquid to be administered to said patient (P) through the gastroenteric apparatus or through a vein.
